# EUROPEAN PATENT APPLICATION

(11) **EP 4 183 338 A1**
(43) Date of publication of application: **24.05.2023**
(21) Application number: 21841434.0
(22) Date of filing: 31.05.2021
(51) Int. Cl.: A61B 5/145, A61B 5/00

(54) **BODY FLUID ANALYTE DETECTION APPARATUS**

(30) Priority: 15.07.2020 WO PCT/CN2020/102017; 03.08.2020 WO PCT/CN2020/106518
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/097169
(87) International publication number: WO 2022/012186

(57) **Abstract**

The invention provides a body fluid analyte detection device, which applies force to the forced part of the bottom shell in one direction to make the bottom shell failed, thereby separating the bottom shell from the transmitter. Before the bottom shell is installed on the human body, the bottom shell is fixed with the installation unit through the clamp part, which reduces the operation steps of the user when separating the bottom shell from the transmitter, reduces the failure rate of the bottom shell before it is installed on the human body, and enhances the user experience, improve the reliability of the body fluid analyte detection device.

## Description

### TECHNICAL FIELD

The invention mainly relates to the field of medical devices, in particular to a body fluid analyte detection device.

### BACKGROUND

The pancreas in a normal human body can automatically monitor the blood glucose level and automatically secrete required amount of insulin/glucagon. In the body of a type 1 diabetes patient, the pancreas does not function properly and cannot produce enough insulin for the body. Therefore, type 1 diabetes is a metabolic disease caused by abnormal pancreatic function, and diabetes is a lifelong disease. At present, there is no cure for diabetes with medical technology. The onset and development of diabetes and its complications can only be controlled by stabilizing blood glucose.

Diabetics need to have their blood glucose measured before they inject insulin into the body. At present, most of the testing methods can continuously measure blood glucose level and transmit the data to a remote device in real time for the user to view. This method is called Continuous Glucose Monitoring (CGM).

When separating the transmitter from the bottom shell, the user needs to apply force in different directions. It requires multiple steps to separate the transmitter from the bottom shell. The user experience is poor.

Therefore, the prior art urgently needs a body fluid analyte detection device that is simple and compact in structure, small in size and convenient for users.

### BRIEF SUMMARY OF THE INVENTION

The invention provides a body fluid analyte detection device, on the one hand, applying force to the forced part of the bottom shell in only one direction can make the bottom shell failed, thus separating the bottom shell from the transmitter. On the other hand, before the bottom shell is installed on the human body, the bottom shell is fixed with the installation unit through the clamp part, which reduces the user's operation steps when separating the transmitter from the bottom shell, and also reduces the failure rate of the bottom shell before it is installed on the human body, enhance the user experience and improve the reliability of the body fluid analyzer detection device.

The invention provides a body fluid analyte detection device, which comprises: a transmitter which is provided with at least one first clamp part. The bottom shell is provided with a second clamp part corresponding to the first clamp part. After the bottom shell is installed on the human body, the first clamp part and the second clamp part are clamped to each other. The transmitter is assembled on the bottom shell. The bottom shell comprises a fixed part and a forced part. When separating the bottom shell and the transmitter, the fixed part is fixed, and the force is applied to the forced part in one direction, the bottom shell fails, at least one pair of first and second clamp parts that are clamped with each other are separated from each other, thereby separating the bottom shell and the transmitter. The sensor comprises a base and a probe, the base is used to fix the sensor and the bottom shell, the probe is used to detect the parameter information of the body fluid analyte, and the sensor is connected with the transmitter to transmit the parameter signal. The battery is used to supply power to the transmitter. The battery is arranged in the bottom shell or the transmitter, and the part for setting the battery is the battery part. The installation unit is provided with a third clamp part corresponding to the second clamp part. Before the bottom shell is installed on the human body, the third clamp part and the second clamp part are clamped to each other, so that the bottom shell is fixed on the installation unit.

According to one aspect of the invention, the side of the bottom shell is provided with an outward convex part, which is a forced part.

According to one aspect of the invention, the battery is arranged in the bottom shell, and at least one connection hole is arranged in the bottom shell. Through the connection hole, the transmitter is electrically connected with the two poles of the battery, and the battery part is the forced part.

According to one aspect of the invention, the transmitter is provided with two first clamp parts, the bottom shell is correspondingly provided with two second clamp parts, and in the bottom shell, the two sides of the connecting line *l₁* of the two second clamp parts are respectively provided with a forced part and a fixed part.

According to one aspect of the invention, a crease groove is arranged on the bottom shell at the position corresponding to the connecting line *l₁.*

According to one aspect of the invention, two second clamp parts are hooks and are arranged on the side wall of the bottom shell.

According to one aspect of the invention, the convex part is arranged on the same side close to the two second clamp parts.

According to one aspect of the invention, the failure mode of the bottom shell comprises one or more combinations of the bottom plate or side wall of the bottom shell fracture, the bottom shell fracture, the second clamp part fracture, and the bottom shell deformation.

According to one aspect of the invention, the bottom shell comprises an adhesive tape for mounting the bottom shell on the human body.

Compared with the prior art, the technical scheme of the invention has the following advantages:
In the body fluid analyte detection device disclosed by the invention, at least one first clamp part is arranged on the transmitter, a second clamp part corresponding to the first clamp part is arranged on the bottom shell, and the bottom shell comprises a fixed part and a forced part. When separating the bottom shell from the transmitter, the fixed part is fixed, and the force is applied to the forced part in one direction. The bottom shell fails, and at least one pair of first clamp part and the second clamp part that clamped together is separated from each other, then the bottom shell and transmitter are separated, the installation unit is provided with a third clamp part corresponding to the second clamp part, and the body fluid analyte detection device is compact in structure and small in size.

Further, before the bottom shell is installed on the human body, the bottom shell is fixed on the installation unit, which saves the step of installing the bottom shell on the installation unit and facilitates the user's installation and use.

Further, the side of the bottom shell is provided with an outward convex part, which is a forced part. Setting the convex part is more conducive to setting the action point of the force, facilitating the failure of the bottom shell, and making it easier to separate the transmitter from the bottom shell.

Further, the battery is arranged in the bottom shell, and at least one connection hole is arranged in the bottom shell. Through the connection hole, the transmitter is electrically connected with the two poles of the battery, and the battery part is the forced part. The battery part is a part of the bottom shell and serves as the forced part. The bottom shell does not need to be set with additional convex parts, which can easily separate the transmitter from the bottom shell and reduce the volume of the detection device. Secondly, because the battery part is thick and the area is relatively large, as the forced part, the user will be easier to implement the separated force and simplify the user's operation steps.

Further, the bottom shell is provided with two second clamp parts, and a crease groove is arranged on the bottom shell at the position corresponding to the connecting line *l₁* of the two second clamp parts. The crease groove can reduce the thickness of the bottom shell at this position. When the force is applied to the forced part, the bottom shell is more likely to fail along the crease groove, making the separation operation easier.

Further, the convex part is arranged on the same side near the two second clamp parts. The position of the convex part is close to that of the two second clamp parts. Applying a small force on the convex part can make the bottom shell failed, which is convenient for users to separate.

Further, before the bottom shell is separated from the installation unit, the sensor is fixed on the installation unit to prevent the sensor probe from being exposed and affecting the use of the probe.

Further, when the bottom shell is separated from the installation unit, the installation unit synchronously installs the sensor on the bottom shell, simplifying the installation steps and facilitating the user's use.

### BRIEF DESCRIPTION OF THE DRAWINGS

Fig. 1 is the sectional structure diagram of the body fluid analyte detection device and its installation unit according to an embodiment of the invention.
Fig. 2 is the three-dimensional structure diagram of a body fluid analyte detection device according to an embodiment of the invention.
Fig. 3 is the three-dimensional structure diagram of the installation unit according to an embodiment of the invention.
Fig. 4 is the schematic diagram of the assembly structure of the transmitter and the bottom shell according to an embodiment of the invention.
Figs. 5a and 5b are the structural diagrams of the side wall or bottom plate of the bottom shell before and after failure according to an embodiment of the invention.
Fig. 5c and Fig. 5d are the structural diagrams before and after the failure of the second clamp part of the bottom shell according to an embodiment of the invention.
Fig. 6 is the schematic diagram of the three-dimensional structure of the detection device according to another embodiment of the invention.
Fig. 7a is the schematic diagram of the assembly structure of the transmitter and the bottom shell according to another embodiment of the invention.
Fig. 7b is the top view of a bottom shell according to another embodiment of the invention.
Fig. 8 is the structural diagram of the detection device according to another embodiment of the invention, which comprises only one first clamp part and one second clamp part respectively.
Fig. 9 is the top view of a detection device including two forced parts according to another embodiment of the invention.

### DETAILED DESCRIPTION

As mentioned above, when installing and separating the transmitter and the bottom shell with the detection device of the prior art, the user needs to apply force in different directions, and needs to operate multiple steps to achieve the installation and separation of the transmitter and the bottom shell. At the same time, the bottom shell is easy to lose effectiveness due to breaking the structure before installation, and the user experience is poor.

In order to solve this problem, the invention provides a body fluid analyte detection device. On the one hand, the force is applied to the forced part of the bottom shell only in one direction, which can make the bottom shell failed, thus separating the first clamp part from the second clamp part. On the other hand, before the bottom shell is installed on the human body, the bottom shell is fixed with the installation unit through the clamp part, which reduces the user's operation steps when separating the transmitter from the bottom shell, it also reduces the failure rate of the bottom shell before it is installed on the human body, enhances the user experience, and improves the reliability of the body fluid analyzer detection device.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the invention.

In addition, it should be understood that, for the convenience of description, the dimensions of each component shown in the drawings are not necessarily drawn according to the actual scale relationship, for example, the thickness, width, length or distance of some elements can be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

### First embodiment

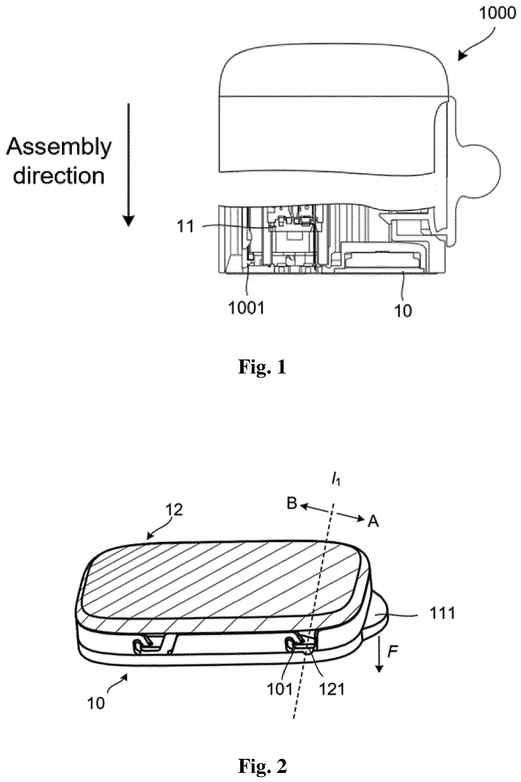

Fig. 1 shows the sectional structure of the body fluid analyte detection device and its installation unit in the embodiment of the invention. Before being installed on the human body, the bottom shell 10 of the body fluid analyzer detection device is fixedly connected with the installation unit 1000.

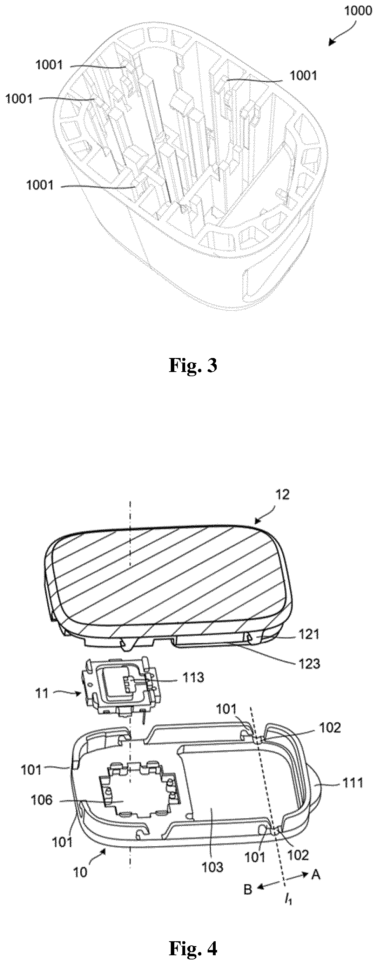

Fig. 2 is the three-dimensional structure diagram of the body fluid analyte detection device in the embodiment of the invention, and Fig. 3 is the three-dimensional structure diagram of the installation unit in the embodiment of the invention.

The detection device comprises the bottom shell 10, the sensor 113 and the transmitter 12.

The bottom shell 10 is used for assembling the transmitter 12 and the sensor 113, and pasting the detection device on the skin surface. The bottom shell 10 comprises a fixed part and a forced part. The bottom shell 10 is provided with at least one second clamp part 101. The second clamp part 101 is used to engage the transmitter 12.

In the embodiment of the invention, the fixed part and the forced part are relative concepts. According to the structural design of the bottom shell 10 and the transmitter 12, the positions of the fixed part and the forced part can be selected differently.

In the embodiment of the invention, the connecting line *li of* the two second clamp parts 101 divides the bottom shell 10 into A side and B side. A side is equipped with a forced part, and B side is equipped with a fixed part.

Therefore, in the embodiment of the invention, the process of separating the bottom shell 10 from the transmitter 12 is as follows: fix the fixed part on the B side with a finger, and apply a force F to the forced part on the A side with another finger in one direction to make the second clamp part 101 failed, and then separate the second clamp part 101 from the first clamp part 121 to separate the transmitter 12 from the bottom shell 10.

In the embodiment of the invention, the forced part is a convex part 111 facing outward from the side of the bottom shell. The shape, size and quantity of the convex part 111 are not limited. Preferably, the convex part 111 is semicircular arc, which is convenient for users to press with their fingers, saves space, and ensures a small and compact bottom shell structure.

In the embodiment of the invention, the number of the second clamp part 101 is two. The two second clamp parts 101 correspond to the side walls of the bottom shell 10.

In other embodiments of the invention, the number of the second clamp parts 101 is four, and four second clamp parts 101 are correspondingly arranged on the opposite side walls of the bottom shell 10, two on each side.

In other embodiments of the invention, the number of the second clamp parts 101 is six, and the six second clamp parts 101 correspond to the side walls of the bottom shell 10, with two on each side.

Here, the fixed part and the forced part are relative concepts. According to the structural design of the bottom shell 10 and the transmitter 12, the positions of the fixed part and the forced part can be selected differently, which will be described in detail below.

The installation unit 1000 is provided with a third clamp part 1001 corresponding to the second clamp part 101, and the second clamp part 101 is connected with the third clamp part 1001 by clamping to fix the bottom shell 10 on the installation unit 1000. When the user fixes the bottom shell 10 on the human body, the installation unit 1000 is also fixed on the human body, which is convenient for the user to operate with one hand.

While the bottom shell 10 is separated from the installation unit 1000, the installation unit 1000 installs the sensor unit 11 on the bottom shell 10, which briefly describes the steps for the user to install the sensor unit 11 on the bottom shell 10. The sensor unit 11 is used to detect the body fluid analyte parameter information.

The transmitter 12 is used for receiving the detection signal generated by the sensor unit 11 and transmitting the signal wirelessly to the remote device. Therefore, transmitter 12 is electrically connected to sensor unit 11.

The transmitter 12 is provided with at least one first clamp part 121. The first clamp part 121 corresponds to the second clamp part 101. The transmitter 12 is assembled on the bottom shell 10 by clamping the second clamp part 101 and the first clamp part 121 to each other. Obviously, in the embodiment of the invention, the transmitter 12 is provided with two first clamp parts 121, that is, two pairs of first clamp parts 121 and second clamp parts 101 that clamp together with each other.

Here, the first clamp part 121 corresponds to the second clamp part 101, which means that they are equal in number and corresponding in position.

When separating the bottom shell 10 from the transmitter 12, the fixed part is fixed by a finger or other devices. When another finger or other auxiliary devices are used to apply force to the forced part in one direction, the bottom shell 10 will become failed. The second clamp part 101 and the first clamp part 121 are separated from each other, thus separating the transmitter 12 from the bottom shell 10. That is, when separating the bottom shell 10 and the transmitter 12, the user can separate them by applying force to the forced part with only one finger in one direction, which is convenient for the user to operate.

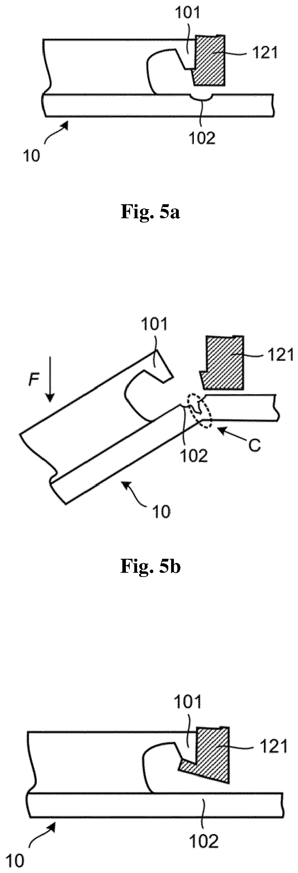

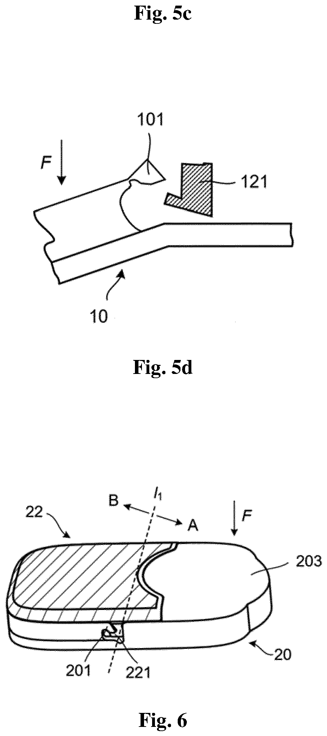

It should be noted here that failure is a conventional concept in the field of engineering materials. After the failure, the material will lose its original function, and the failed part cannot be restored again. Since the second clamp part 101 is a part of the bottom shell 10, the failure of the bottom shell 10 comprises the failure of the bottom plate, side wall or the second clamp part 101 of the bottom shell 10. Therefore, the failure mode of the bottom shell 10 comprises one or more of the bottom plate or side wall fracture of the bottom shell 10 (as shown in Fig. 5b), the bottom shell 10 fracture, the second clamp part 101 fracture (as shown in Fig. 5d), and the plastic deformation of the bottom shell 10. Obviously, after the bottom shell 10 fails, the bottom shell 10 will lose the function and function of engaging the transmitter 12.

The methods for fixing the fixed part comprise clamping, supporting, etc. There are no specific restrictions here, as long as the conditions for fixing the fixed part can be met.

Specifically, in the embodiment of the invention, the connecting line *l₁* of the two second clamp parts 101 divides the bottom shell 10 into A side and B side. A side is provided with a forced part, and B side is provided with a fixed part. Since the outer contour of the transmitter 12 basically coincides with that of the bottom shell 10, the side of the bottom shell 10 in the embodiment of the invention is provided with an outward convex part 111, which is convenient for applying force. The convex part 111 is the forced part.

The convex part 111 is arranged on the short side of the bottom shell 10, and the two second clamp parts 101 are correspondingly arranged on the long side of the bottom shell 10. The two second clamp parts 101 and the two first clamp parts 121 are hooks. When separating the transmitter 12 from the bottom shell 10, the convex part 111 in the embodiment of the invention is arranged on the same side near the two second clamp parts 101, as shown in Fig. 1. The convex part 111 is relatively close to the two second clamp parts 101. Applying a small force on the convex part 111 can make the second clamp part 101 failed, which is convenient for users to separate.

Therefore, in the embodiment of the invention, the process of separating the bottom shell 10 from the transmitter 12 is as follows: fix the fixed part on the side B with a finger, and apply a force F to the convex part 111 in one direction with another finger to make the second clamp part 101 failed, and then separate the second clamp part 101 from the first clamp part 121, so as to separate the transmitter 12 from the bottom shell 10.

It should be noted that the embodiment of the invention does not limit the positions of the convex part 111 and the second clamp part 101. For example, the convex part 111 can be arranged on the long side of the bottom shell 10, and the two second clamp parts 101 are correspondingly arranged on the short side. Or the convex part 111 is far away from the second clamp part 101, so applying a greater force on the convex part 111 can also make the second clamp part 101 ineffective and separate from the first clamp part 121. Or the second clamp part 101 can also be arranged on the bottom plate of the bottom shell 10, and there is no specific restriction here.

In the embodiment of the invention, the shape of the top view of the detection device is a rounded rectangle. The embodiment of the invention does not specifically limit the shape of the top view of the detection device, and its shape can also be rectangular, circular, elliptical or other shapes.

Fig. 4 shows the assembly structure of transmitter 12 and bottom shell 10 in the embodiment of the invention.

The detection device also comprises a battery (not shown). The battery is used to power the transmitter. The position where the battery is set is the battery position 123. Specifically, in the embodiment of the invention, the battery is set in the transmitter 12. The battery is set in the transmitter 12, which can be charged for many times and reused to reduce the cost of users.

Since the battery has a certain volume, the bottom shell 10 of the embodiment of the invention is also provided with an assembly hole 103 for assembling the battery part 123. Specifically, in the embodiment of the invention, after the battery part 123 is installed to the assembly hole 103, part of the battery part 123 is exposed outside the bottom shell 10. And the convex part 111 is arranged on one side of the assembly hole 103. Therefore, when a force F is applied to the convex part 111 to separate the bottom shell 10 from the transmitter 12, the exposed battery part 123 can be used as a support part for applying the force F (for example, the thumb is supported on the exposed battery part 123). At this time, it is equivalent to that the user's thumb exerts a force opposite to the force F on the transmitter 12 to promote the separation of the bottom shell 10 from the transmitter 12.

It should be noted that in other embodiments of the invention, the assembly hole 103 may not be set, that is, the battery part 123 is completely wrapped by the bottom shell 10 and is not exposed.

In the embodiment of the invention, a crease groove 102 is also arranged at the position corresponding to the connecting line 11 on the bottom shell 10. The crease groove 102 can reduce the thickness of the bottom shell. The bottom shell 10 is more likely to fail along the crease groove 102, and the separation operation is easier. Due to the assembly hole 103, the crease groove 102 is located on the side wall of the bottom shell 10. It is obvious that the crease groove 102 can penetrate the bottom plate of the bottom shell 10 along the connecting line *l₁* without the assembly hole 103.

Preferably, in the embodiment of the invention, the bottom plate of the bottom shell 10 is also provided with a mounting hole 106 and a sensor unit 11 arranged in the mounting hole 106. The edge contour of the sensor unit 11 matches the edge contour of the mounting hole 106. Here, the matching of the two edge contours means that the two edges can fit into each other.

One part of the sensor 113 is arranged on the sensor unit 11, and the other part enters the subcutaneous area. When installing the sensor 113, the sensor 113 is loaded by the sensor unit 11 and installed on the chassis 10. Preferably, in the embodiment of the invention, before and after installation, the shape of the sensor 113 on the sensor unit 11 does not change, that is, the sensor 113 and the sensor unit 11 are simultaneously installed on the bottom shell 10. Therefore, after installing the sensor 113, the sensor unit 11 becomes part of the bottom shell 10.

Fig. 5a and Fig. 5b are the structural diagrams of the side wall or bottom plate of the bottom shell 10 before and after failure. Fig. 5c and Fig. 5d are the structural diagrams of the second clamp part 101 of the bottom shell 10 before and after failure.

As previously mentioned, the first clamp part 121 and the second clamp part 101 are hooks. When a force F is applied to the forced part in one direction, the bottom shell 10 is broken along the crease groove 102, as shown by the virtual coil C. In Fig. 5c - Fig. 5d, when the forced part is applied with force F in one direction, the second clamp part 101 is broken.

In other embodiments of the invention, the first clamp part 121 and the second clamp part 101 can be respectively a card hole and a card block, or a card block and a card slot, and no specific restriction is made here.

### Second embodiment

The difference between the second embodiment and the first embodiment is that the battery is arranged on the bottom shell and there is no assembly hole. Other structures and connection modes are similar to those in the first embodiment.

Fig. 6 is the three-dimensional structure diagram of the detection device in the embodiment of the invention.

In the embodiment of the invention, the battery is arranged in the bottom shell 20. Therefore, the battery part 203 is located on the bottom shell 20. The top of the battery part 203 is flush with the top of the transmitter 22, which can reduce the thickness size of the detection device. At this time, the bottom shell 20 is not provided with a convex part, but the battery part 203 is directly used as the forced part. As the battery part 203 is thicker and has a relatively large area, as a forced part, it is easier for users to apply the force on the battery part, optimizing the user's operation steps.

Similarly, two second clamp parts 201 are arranged on both sides of the bottom shell 20. Therefore, the third clamp part 1001 corresponding to the installation unit 1000 is similar to the first embodiment. It can be understood by those skilled in the art that when the number, position, size and other parameters of the second clamp part in the second embodiment change with respect to the second clamp part 101 in the first embodiment, the third clamp part 1001 in the second embodiment will also change accordingly, so as to be able to engage with the second clamp part.

The connecting line *l₁* of the two second clamp parts 201 divides the bottom shell into A side and B side. The A side is provided with the battery part 203, which is used as the forced part, and the B side is used as the fixed part. When separating the bottom shell 20 from the transmitter 22, fix the fixed part and apply force F to the battery part 203 in one direction to make the bottom shell 20 failed, thus separating the transmitter 22 from the bottom shell 20.

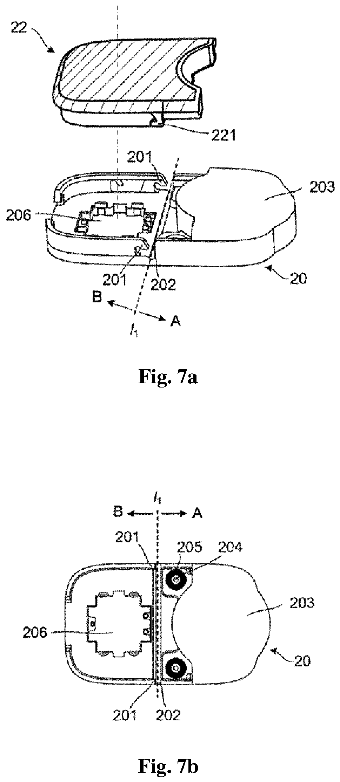

Fig. 7a shows the assembly structure of the transmitter 22 and the bottom shell 20 in the embodiment of the invention. Fig. 7b is a top view of the bottom shell 20 in the embodiment of the invention.

As shown in Figs. 7a and 7b, the crease groove 202 corresponding to the connecting line *l₁* penetrates the bottom plate of the bottom shell 20. When the first clamp part 221 is separated from the first clamp part 201, the bottom shell 20 is broken along the crease groove 202.

Since the battery needs to supply power to the transmitter 22, the bottom shell 20 is also provided with at least one connection hole 204. The transmitter 22 is electrically connected with the anode and cathode electrodes of the battery through the connection hole 204.

The connection hole 204 can be provided with an electrical contact which can be electrically connected with the transmitter 22. Or the transmitter 22 is provided with a protruding electrical connection end that can be inserted into the connection hole 204. Or the battery can be electrically connected with the transmitter 22 through the wire passing through the connection hole 204 or the lead plated on the surface of the bottom shell 20.

It should be noted that in other embodiments of the invention, the connection hole 204 may not be set, and the transmitter 22 and the two poles of the battery are electrically connected through the lead wire completely coated on the surface of the bottom shell 20, which is not specifically limited here.

Preferably, in the embodiment of the invention, in order to seal the electrical connection position, a sealing ring 205 is arranged around the connection hole 204.

In general, the elastic sealing material plays a sealing role after being squeezed. In the embodiment of the invention, the extruded seal ring 205 exerts certain elastic force on the transmitter 22. When applying force F to the forced part, the sealing ring 205 provides the elastic force to promote the separation of the transmitter 22 from the bottom shell 20.

Preferably, in the embodiment of the invention, in order to seal the connection position between the transmitter 22 and the sensor, a seal (not shown) is arranged around the sensor. Similar to the above seal ring 205, when applying force F to the forced part, the seal also provides the elastic force to promote the separation of the transmitter 22 from the bottom shell 20.

It should be noted that since the electrical connection position between the transmitter 22 and the battery or the connection position between the transmitter 22 and the sensor is relatively good, other embodiments of the invention may not be provided with a seal ring 205 or a seal.

For the failure mode of the bottom shell 20, the function of the mounting hole 206, the type and engagement mode of the first clamp part 221 and the second clamp part 201, please refer to the previous description, and will not be repeated here.

In other embodiments of the invention, the transmitter 22 part mounted on the bottom shell 20 can also be a forced part, which is not specifically limited here. It should be pointed out here that although the force F may directly act on the transmitter 22, the actual effect of the force F is only displayed on the bottom shell 20, that is, the force F only changes the shape of the bottom shell 20 (such as shape and structure changes), but does not change the shape of the transmitter 22. At this time, it can still be considered that the user is the force exerted on the forced part of the bottom shell 20.

### Third embodiment

Compared with the previous embodiment, the third embodiment is different in that only one first clamp part is set on the transmitter, only one second clamp part is correspondingly set on the bottom shell, and the shape of the top view of the detection device is oval.

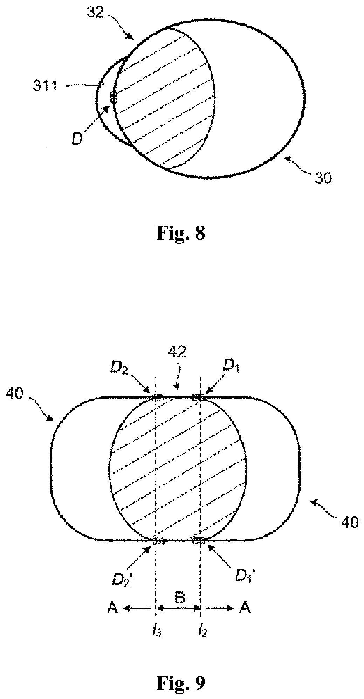

Fig. 8 is the structural diagram of the detection device according to the embodiment of the invention, which only comprises a first clamp part and a second clamp part.

A first clamp part and a second clamp part are engaged with each other at D, that is, there is only one pair of first clamp parts and second clamp parts engaged with each other. The bottom shell 30 comprises a convex part 311. The convex part 311 is a forced part, and the rest of the bottom shell 30 is a fixed part. By applying force to the convex part 311, the bottom shell 30 fails, and the first clamp part and the second clamp part are separated from each other, thus separating the transmitter 32 from the bottom shell 30.

For the failure mode of the bottom shell 30, the types and engagement modes of the first clamp part and the second clamp part, please refer to the above, and will not be repeated here.

It can be understood by those skilled in the art that the installation unit 1000 is also provided with a third clamp part corresponding to the second clamp part. When the number, position, size and other parameters of the second clamp part in the third embodiment change with respect to the second clamp part in the first embodiment, the third clamp part in the third embodiment will also change accordingly to engage with the second clamp part.

### Fourth embodiment

Compared with the previous embodiment, the fourth embodiment is different in that the detection device comprises a plurality of forced parts.

It can be understood by those skilled in the art that the change in the implementation mode of the forced part structure does not cause the clamping structure and the clamping relationship of the first clamp part, the second clamp part and the third clamp part, so it will not be repeated here.

Fig. 9 is a top view of the detection device of the embodiment of the invention, which comprises two forced parts.

In the embodiment of the invention, the transmitter 42 is provided with four first clamp parts, and the bottom shell 40 is also correspondingly provided with four clamp parts. The two corresponding first clamp parts and the second clamp parts engage with each other at D1-D1' and D2-D2' respectively, that is, four pairs of first clamp parts and second clamp parts engage with each other. On the bottom shell 40, the connecting lines of D1-D1' and D2-D2' are *l₂* and *l₃* respectively. The A side and B side of *l₂* and *l₃* are respectively provided with a force applying part and a fixed part. *l₂* and *l₃* divide the bottom shell 40 into a forced part, a fixed part and a forced part. In the embodiment of the invention, two forced parts are arranged on both sides of the bottom shell 40, and a fixed part is arranged between the two forced parts.

When separating the transmitter 42 and the bottom shell 40, two thumbs are supported below the fixed part to fix the fixed part, and two index fingers apply force to the forced part in one direction on both sides of the detection device, making the bottom shell 40 more likely to fail.

For the failure mode of the bottom shell 40, the types and engagement modes of the first clamp part and the second clamp part, please refer to the above, and will not be repeated here.

It should be noted that other embodiments of the invention can also be provided with more first and second clamp parts. When separating the transmitter from the bottom shell, at least one pair of first and second clamp parts fail (similar to the way shown in Fig. 8), or two pairs of corresponding first and second clamp parts fail (similar to the way shown in Fig. 4 and Fig. 7a), or multiple pairs of corresponding first and second clamp parts fail (similar to the way shown in Fig. 9).

To sum up, the invention discloses a body fluid analyte detection device. On the one hand, before the bottom shell is installed on the human body, the bottom shell is fixed with the installation unit, and the sensor is located in the installation unit and separated from the bottom shell. When the bottom shell is separated from the installation unit, the installation unit simultaneously installs the sensor on the bottom shell, reducing the steps of installing the sensor on the bottom shell. On the other hand, the installation unit only applies force to the forced part of the bottom shell in one direction, the utility model can make the bottom shell failed, thereby separating the first clamp part from the second clamp part, reducing the user's operation steps when separating the transmitter from the bottom shell, and enhancing the user's experience.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope and spirit of the invention. The scope of the invention is limited by the attached claims.

## Claims

1. A body fluid analyte detection device is **characterized by**, comprising:
a transmitter which is provided with at least one first clamp part;
the bottom shell is provided with a second clamp part corresponding to the first clamp part. After the bottom shell is installed on the human body, the first clamp part and the second clamp part are clamped to each other. The transmitter is assembled on the bottom shell. The bottom shell comprises a fixed part and a forced part. When separating the bottom shell and the transmitter, the fixed part is fixed, and the force is applied to the forced part in one direction, the bottom shell fails, at least one pair of first and second clamp parts that are clamped with each other are separated from each other,
thereby separating the bottom shell and the transmitter;
the sensor comprises a base and a probe, the base is used to fix the sensor and the bottom shell, the probe is used to detect the parameter information of the body fluid analyte, and the sensor is connected with the transmitter to transmit the parameter signal;
the battery is used to supply power to the transmitter. The battery is arranged in the bottom shell or the transmitter, and the part for setting the battery is the battery part;
the installation unit is provided with a third clamp part corresponding to the second clamp part. Before the bottom shell is installed on the human body, the third clamp part and the second clamp part are clamped to each other, so that the bottom shell is fixed on the installation unit.

2. Body fluid analyte detection device of claim 1, **characterized in that**, the side of the bottom shell is provided with an outward convex part, which is a forced part.

3. Body fluid analyte detection device of claim 1, **characterized in that**, the battery is arranged in the bottom shell, and at least one connection hole is arranged in the bottom shell. Through the connection hole, the transmitter is electrically connected with the two poles of the battery, and the battery part is the forced part.

4. Body fluid analyte detection device of claim 2, **characterized in that**, the transmitter is provided with two first clamp parts, the bottom shell is correspondingly provided with two second clamp parts, and in the bottom shell, the two sides of the connecting line *l₁* of the two second clamp parts are respectively provided with a forced part and a fixed part.

5. Body fluid analyte detection device of claim 4, **characterized in that**, a crease groove is arranged on the bottom shell at the position corresponding to the connecting line /*₇.*

6. Body fluid analyte detection device of claim 4, **characterized in that**, two second clamp parts are hooks and are arranged on the side wall of the bottom shell.

7. Body fluid analyte detection device of claim 4, **characterized in that**, the convex part is arranged on the same side close to the two second clamp parts.

8. Body fluid analyte detection device of claim 1, **characterized in that**, the failure mode of the bottom shell comprises one or more combinations of the bottom plate or side wall of the bottom shell fracture, the bottom shell fracture, the second clamp part fracture, and the bottom shell deformation.

9. Body fluid analyte detection device of claim 1, **characterized in that**, the bottom shell comprises an adhesive tape for mounting the bottom shell on the human body.
